# EUROPÄISCHE PATENTANMELDUNG

(11) **EP 2 628 479 A1**
(43) Veröffentlichungstag der Anmeldung: **21.08.2013**
(21) Anmeldenummer: 12191357.8
(22) Anmeldetag: 06.11.2012
(51) Int. Cl.: A61K 8/49, A61Q 19/08, A61Q 19/00, A61Q 19/02

(54) **Kosmetische Verwendung von Climbazol**

(30) Priorität: 29.11.2011 DE 102011087319
(71) Anmelder: Henkel AG&Co. KGAA, 40589 Düsseldorf (DE)
(72) Erfinder: Holtkötter, Olaf, 50354 Hürth (DE); Janßen, Frank, 41470 Neuss (DE); Welß, Thomas, 40591 Düsseldorf (DE); Kippenberger, Stefan, 60316 Frankfurt am Main (DE); Heinen, Gudrun, 40215 Düsseldorf (DE); Schuh, Werner, 40223 Düsseldorf (DE)

(57) **Zusammenfassung**

Die Erfindung betrifft die kosmetische, nicht-therapeutische Verwendung von Climbazol (Imidazolyl-1-(4-chlorophenoxy)-3,3-dimethylbutane-2-one) zur Beeinflussung der natürlichen Produktion von Bestandteilen in der Dermis.

Die Erfindung betrifft weiterhin ein kosmetisches, nicht-therapeutisches Verfahren zur Verbesserung der Hautfestigkeit und/oder zur Hautstraffung und/oder zur Reduktion von Hautfalten, bei dem Climbazol in einem geeigneten kosmetischen Träger mit der Haut in Kontakt gebracht wird, sowie ein kosmetisches, nicht-therapeutisches Verfahren zur Verbesserung dermalen Gewebeschwundes, bei dem Climbazol in einem geeigneten kosmetischen Träger mit der Haut in Kontakt gebracht wird.

## Beschreibung

Die Erfindung liegt auf dem Gebiet der Kosmetik und betrifft die kosmetische, nicht-therapeutische Verwendung von Climbazol zur Beeinflussung der natürlichen Produktion von Bestandteilen in der Dermis, sowie kosmetische Verfahren zur Verbesserung der Hautfestigkeit und zur Verbesserung dermalen Gewebeschwundes.

Die positiven Wirkungen von Retinol und/oder Retinoiden in kosmetischen - insbesondere topischen - und/oder pharmazeutischen Zusammensetzungen sind weithin bekannt.
Beispielsweise wurde nachgewiesen und beschrieben, dass Retinsäure und/oder Retinol in der Dermis die Neuproduktion von Bestandteilen der extrazellulären Matrix, beispielsweise des Kollagens, stimulieren, und in der Epidermis die Proliferation und Differenzierung der Keratinocyten regulieren kann (können).
Makroskopisch führt die Verwendung von Retinsäure und/oder Retinol in topischen Zusammensetzungen zu einem verfeinerten Hautbild, einer Aufhellung von Pigment- und/oder Altersflecken, zu einer Normalisierung der Epidermisdicke sowie zu einer Straffung der Haut.
In pharmazeutischen Zusammensetzungen werden Retinol und Retinoide aufgrund ihrer hohen Wirksamkeit in der Behandlung gegen Verhornungsstörungen der Haut, wie beispielsweise Fischschuppenkrankheit (Ichthyosis) und/oder Schuppenflechte (Psoriasis), oder in der Behandlung starker Akne verwendet.

Problematisch bei der Verwendung von Retinoiden - insbesondere in kosmetischen Zusammensetzungen - sind die starken Nebenwirkungen der Retinoide (Teratogenität, Hypertriglyceridaemie und/oder Retinoide Dermatitis).
Diese unerwünschten Nebenwirkungen schließen eine Verwendung von Retinoiden im kosmetischen Massenmarkt nahezu aus.

In der Vergangenheit war man deshalb bemüht, wirksame Alternativen für Retinoide zu finden, die die gleichen positiven Eigenschaften wie Retinoide aufweisen, und die gleichzeitig die zuvor genannten, negativen Eigenschaften der Retinoide nicht aufweisen.

Als eine Klasse möglicher Alternativen für Retinoide wurden die sogenannten RAMBAs (Retinoic Acid Metabolism Blocking Agents) vorgeschlagen.
RAMBAs wurden bislang für pharmazeutische Anwendungen beschrieben (Verfaille et al. (2008), JDDG 5:355-364), insbesondere aufgrund ihrer Wirkung, Enzyme zu blockieren (vorwiegend solche Enzyme, die für den Abbau zellulärer Retinsäure verantwortlich sind, speziell Cytochrom C-Enzym Cyp26A1).

Bislang bekannte RAMBAs sind jedoch meist nicht kosmetisch nutzbar, da ein Großteil der bekannten RAMBAS für die kosmetische Verwendung nicht zugelassen ist.

Es ist daher erstrebenswert Wirkstoffe und/oder Wirkstoffkombinationen zu finden, die bereits für die kosmetische Verwendung zugelassen sind, und die eine RAMBA-Aktivität aufweisen.

Climbazol (Imidazolyl-1-(4-chlorophenoxy)-3,3-dimethylbutane-2-one) wird in kosmetischen Haarbehandlungsmitteln seit langem als wirksames Antischuppenmittel eingesetzt.
Darüber hinaus wurde Climbazol im Stand der Technik auch als Wirkstoff für die Hautbehandlung beschrieben:
In der Anmeldung US 5582832 wird eine Zusammensetzung zur Behandlung faltiger und/oder UVlichtgeschädigter Haut offenbart, die eine Kombination eines Azols (Climbazol) und eines Lipids (Ceramid) offenbart.
In den Anmeldungen WO 2002/053089 und WO 2002/053124 werden hautpflegende Emulsionen beansprucht, die ein Retinoid und einen Retinoid-Booster enthalten. Climbazol wir als geeigneter Retinoid-Booster genannt.
US 5716627 offenbart ein Hautkonditionierungsmittel, das Retinol, ein Azol (beispielsweise Climbazol) und ein Fettsäureamid enthält, welches einen positiven Einfluss auf die Proliferation und Differenzierung der Keratinocyten ausübt.

In keinem der zuvor genannten Dokumente wird eine RAMBA-Wirkung von Climbazol beschrieben.

Der vorliegenden Erfindung lag die Aufgabe zugrunde, Wirkstoffe und/oder Wirkstoffkombinationen zu finden, die sich für die Verwendung in kosmetischen Mitteln eignen, und die eine RAMBA-Aktivität aufweisen.
Insbesondere sollte der Wirkstoff oder die Wirkstoffkombination für die topische Anwendung geeignet sein, und einen positiven Einfluss auf die Kollagenneusynthese in der Dermis haben.

Diese Aufgaben konnten erfüllt werden durch die kosmetische Verwendung von Climbazol.

Gegenstand der vorliegenden Erfindung ist demnach die kosmetische, nicht-therapeutische Verwendung von Climbazol (Imidazolyl-1-(4-chlorophenoxy)-3,3-dimethylbutane-2-one) zur Beeinflussung der natürlichen Produktion von Bestandteilen in der Dermis.

Die "Dermis" ist ein komplexes System aus filamentreichem Bindegewebe, welches mit einem dichten Netz aus Nerven und Blutgefäßen durchzogen ist. Sie umfasst neben elastischen Fasern überwiegend ein enges Geflecht aus (größtenteils) Kollagenfasern, die der Haut Stabilität verleihen. Die Dermis umfasst weiterhin die sogenannte extrazelluläre Matrix, worunter Substanzen und Flüssigkeiten in den Zellzwischenräumen verstanden werden.

Fibroblasten sind fixierte Bindegewebszellen in der Dermis, in denen die Substanzen der extrazellulären Matrix produziert werden.

Die Extrazellulärmatrix (EZM) besteht aus faserigen Bestandteilen sowie aus Flüssigkeit, in der diverse Substanzen gelöst vorliegen. EZM enthält - neben Wasser - überwiegend Glycoproteine und Polysaccharide (Glycosaminglycane) sowie Aminosäuren, Glucose, Gewebshormone und Elektrolytsalze.
Unter den Proteinen enthält die EZM vorwiegend Kollagen sowie elastische Fasern, die aus den Proteinen Fibrillin und Elastin gebildet werden.
Nimmt der Gehalt an Elastin und Kollagen in der EZM ab, wird die Haut teigig, schlaff und faltig. Darüber hinaus verliert sie ihre Elastizität.

Unter "Beeinflussung der natürlichen Produktion von Bestandteilen in der Dermis" wird bevorzugt die Beeinflussung der natürlichen Produktion von Bestandteilen der extrazellulären Matrix in der Dermis verstanden, wobei die Beeinflussung sowohl positiv als auch negativ, bevorzugt positiv, sein kann.
Insbesondere bevorzugt ist die Stimulierung der natürlichen Produktion von Bestandteilen der extrazellulären Matrix.

In einer ersten bevorzugten Ausführungsform der Erfindung wird die natürliche Produktion - insbesondere die Neuproduktion - von Elastin, Kollagen und/oder Glycosaminglykanen in der extrazellulären Matrix durch die kosmetische Verwendung von Climbazol beeinflusst, bevorzugt stimuliert und/oder verbessert.

Glycosaminglycane sind saure Polysaccharide, die linear aus sich wiederholenden Disacchariden aufgebaut sind. Die Disaccharid-Einheiten bestehen meist aus einer Uronsäure, die 1,3-glycosidisch mit einem Aminozucker (beispielsweise N-Acetylglykosamin) verbunden ist. Die Disaccharid-Einheiten sind 1,4-glycosidisch miteinander verknüpft und können teilweise mit Essigsäure oder Schwefelsäure weiter verestert sein.
Je nach Zusammensetzung der Disaccharid-Einheiten unterscheidet man verschiedene Untergruppen der Glycosaminglykane wie Hyaluronsäure, Heparin, Chondroitinsulfat und Keratansulfat.
Hyaluronsäure verbessert das Wasserbindungsvermögen in der Haut. Ein verminderter Hyaluronsäuregehalt in der Haut macht sich bemerkbar durch schlaffe, faltige und/oder trockene Haut.

In einer besonders bevorzugten Ausführungsform wird die natürliche Kollagensynthese in der extrazellulären Matrix durch die kosmetische Verwendung von Climbazol stimuliert und/oder verbessert.

In einer weiteren besonders bevorzugten Ausführungsform wird die natürliche Hyaluronsäuresynthese in der extrazellulären Matrix durch die kosmetische Verwendung von Climbazol stimuliert und/oder verbessert.

Gemäß einer besonderen Ausführungsform erfolgt die erfindungsgemäße Verwendung topisch, d.h. durch Auftragen auf die Haut und/oder Hautanhangsgebilde, bevorzugt durch Auftragen auf die Gesichtshaut, die Kopfhaut und/oder auf die Hautregionen des Halses, des Dekolletes, der Arme und/oder der Beine, insbesondere durch Auftragen auf die Gesichtshaut.

Erfindungsgemäß besonders bevorzugt ist daher weiterhin die kosmetische Verwendung von 0,001 bis 2 Gew.-%, bevorzugt von 0,0025 bis 1,5 Gew.-%, und insbesondere von 0,005 bis 1 Gew.-% Climbazol in einem kosmetischen Mittel, insbesondere in einem kosmetischen Hautbehandlungsmittel, wobei sich die Mengenangaben auf das Gesamtgewicht des kosmetischen Mittels beziehen.

Die kosmetischen Mittel, in denen Climbazol bevorzugt verwendet wird (im Folgenden als "erfindungsgemäß verwendete Mittel" bezeichnet) zeigen über die Beeinflussung der natürlichen Produktion von Bestandteilen in der Dermis hinaus verbesserte Pflegeeffekte auf der Haut und/oder den Hautanhangsgebilden.
Besonders auf der Haut sind die positiven Effekte deutlich ausgeprägt, so dass bevorzugte erfindungsgemäß verwendete kosmetische Mittel Hautbehandlungsmittel sind.

Hautbehandlungsmittel im Sinne der vorliegenden Erfindung können in verschiedenen Angebotsformen konfektioniert werden, beispielsweise als (ggf. öl- und fettfreies) Gel, als Creme, in Stiftform, als flüssige oder gelförmige Roll-on-Applikation, als getränktes flexibles Substrat (Pad), aber auch als Puder oder Spray.

Hautbehandlungsmittel im Sinne der vorliegenden Erfindung können in fester, halbfester, flüssiger, disperser, emulgierter, suspendierter, oder gelförmiger Form vorliegen. Weiterhin können sie als Aerosol konfektioniert sein, das heißt, sie sind in einem Druckbehälter verpackt, aus dem sie mit Hilfe eines Treibmittels versprüht werden können. Weiterhin können die Hautbehandlungsmittel als treibgasfreies Pumpspray versprüht werden.

Bevorzugte Hautbehandlungsmittel im Sinne der vorliegenden Erfindung können beispielsweise Reinigungsmittel für die Haut wie Dusch- und Waschgele und/oder Seifen, Hautpflegemittel wie Lotionen, Gele und/oder Cremes für den Körper und/oder das Gesicht, Gesichtswässer, Lippenpflegemittel, aber auch dekorative kosmetische Mittel wie Make-ups sein.

Besonders bevorzugte Hautbehandlungsmittel sind dadurch gekennzeichnet, dass sie als Hautpflegemittel, insbesondere als Hautpflegemittel für die Gesichtshaut, wie eine Gesichtscreme, ein Gesichtsgel und/oder ein Gesichtswasser, konfektioniert sind.

In einer weiteren bevorzugten Ausführungsform enthalten erfindungsgemäß verwendete Mittel zur Erzielung verbesserter Pflegeeffekte auf der Haut zusätzlich zu Climbazol bevorzugt einen geeigneten kosmetischen Träger, vorzugsweise einen topischen Träger, der weiterhin mindestens einen Wirkstoff, ausgewählt aus
- Harnstoff-Derivaten, ausgewählt aus Verbindungen gemäß Formel (I) 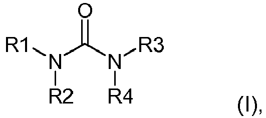 worin
   die Reste R1, R2, R3 und R4 unabhängig voneinander für ein Wasserstoffatom, eine C₁-C₄-Alkylgruppe, eine C₂-C₆-Alkenylgruppe oder eine C₂-C₆-Hydroxyalkylgruppe mit mindestens einer Hydroxygruppe steht,
   mit der Maßgabe, dass mindestens einer der besagten Reste eine C₂-C₆Hydroxyalkylgruppe mit mindestens einer Hydroxygruppe bedeutet,
- Vitaminen, Provitaminen und Vitaminvorstufen der Gruppen B, C, E, H und K und den Estern der vorgenannten Substanzen,
- Monomeren, Oligomeren und Polymeren von Aminosäuren, N-C₂-C₂₄-Acylaminosäuren, den Estern und/oder den physiologisch verträglichen Metallsalzen dieser Substanzen,
- DNA- oder RNA-Oligonucleotiden,
- natürlichen Betainverbindungen,
- α-Hydroxycarbonsäuren, α-Ketocarbonsäuren, β-Hydroxycarbonsäuren und deren Ester-, Lacton- oder Salzform, ausgenommen Zusammensetzungen mit 5 Gew.-% (2-Hydroxyethyl)harnstoff und 0,05 bzw. 5 Gew.-% Ammoniumlactat und Zusammensetzungen mit (2-Hydroxypropyl)harnstoff und α- und/oder β-Hydroxycarbonsäuren,
- den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid,
- Isoflavonoiden und Isoflavonoid-reichen Pflanzenextrakten,
- Ubichinon und Ubichinol sowie deren Derivaten,
- Silymarin,
- natürlich vorkommenden Xanthin-Derivaten, ausgewählt aus Coffein, Theophyllin, Theobromin und Aminophyllin,
- Ectoin,
- Kreatin,
- Olivenblattextrakten, Ursolsäure, Oleanol und/oder Oleanolsäure,
- Mono- und Polyhydroxystilbenen und deren Estern,
- Derivaten von methyliertem Silanol,
- Phytinsäure,
- Extrakten aus Maiskörnern (Zea Mays (Corn) Kernel Extract),
- Extrakten aus Haferkörnern (Avena Sativa (Oat) Kernel Extract),
- Saccharomyces/Xylinum/Black Tea Ferment,
- Apfelkernextrakten (Pyrus Malus (Apple) Fruit Extract),
- Lotuskeim-Extrakten (Nelumbo Nucifera Germ Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Wirkstoffen, die die beta-Endorphinsynthese in Keratinozyten stimulieren,
- anorganischen und organischen UV-Filtersubstanzen,
- selbstbräunenden Wirkstoffen,
- hautaufhellenden Wirkstoffen,
- Wirkstoffen, die die Prostaglandinsynthese und/oder die Leukotrien-Synthese inhibieren,
- sebumregulierenden Wirkstoffen,
- sowie Desoxyzuckern oder Desoxyzucker-Bausteine enthaltenden Polysacchariden, enthält.

Unter einem geeigneten kosmetischen Träger, bevorzugt einem topischen Träger, wird bevorzugt ein wässriger oder wässrig-alkoholischer Träger verstanden.

Bevorzugt enthält der Träger - bezogen auf sein Gesamtgewicht - mindestens 30 Gew.-%, mehr bevorzugt mindestens 35 Gew.-% besonders bevorzugt mindestens 40 Gew.-% und insbesondere mindestens 45 Gew.-% Wasser.

Weiterhin kann der kosmetische Träger 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, 1-Propanol, 2-Propanol, Isopropanol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycole, Sorbitol, Sorbitan, Benzylalkohol, Phenoxyethanol oder Mischungen dieser Alkohole.
Bevorzugt sind die wasserlöslichen Alkohole.
Besonders bevorzugt sind Ethanol, 1-Propanol, 2-Propanol, 1,2-Propylenglycol, Glycerin, und/oder 1,6-Hexandiol sowie Mischungen dieser Alkohole. Insbesondere bevorzugt sind Glycerin und/oder 1,6-Hexandiol.

Besonders bevorzugte Harnstoffderivate der Formel (I) sind solche, die mindestens eine C₂-C₆-Hydroxyalkylgruppe mit mindestens einer Hydroxygruppe enthalten, welche aus mindestens einem

Vertreter aus der Gruppe aus 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxybutyl, 1-Hydroxy-2-methylprop-2-yl, 2,3,4,5,6-Pentahydroxyhexyl und 1,3,4,5,6-Pentahydroxyhex-2-yl ausgewählt wird.

Es ist bevorzugt solche Harnstoffderivate der Formel (I) zu verwenden, welche die bevorzugte Maßgabe der Formel (I) erfüllen, dass mindestens einer der Reste R1 bis R4 eine C₂-C₆-Hydroxyalkylgruppe mit mindestens einer Hydroxygruppe bedeutet. Weiterhin besonders bevorzugt stehen jeweils zwei Reste aus R1, R2, R3 und R4 für eine C₂-C₆-Hydroxyalkylgruppe mit mindestens einer Hydroxygruppe. Insbesondere bevorzugt stehen die zwei Reste aus R1, R2, R3 und R4 für eine C₂-C₆-Hydroxyalkylgruppe mit mindestens einer Hydroxygruppe, wobei diese beiden Reste an unterschiedlichen Stickstoffatomen des Harnstoffs positioniert (N,N'-Stellung) sind. Beispiele für in Verbindungen der Formel (I) verwendbare C₁-C₄-Alkylgruppen sind Methyl, Ethyl, n-Propyl, Isopropyl, n-Butyl, sec-Butyl, Isobutyl und tert-Butyl. Beispiele für in Verbindungen der Formel (I) verwendbare C₂-C₆-Alkenylgruppen sind Vinyl, 2-Propen-1-yl (Allyl) oder 3-Buten-1-yl.

Besonders bevorzugt wird mindestens eine Verbindung aus der Gruppe mit den Vertretern N-(2-Hydroxyethyl)harnstoff, N,N-Bis-(2-hydroxyethyl)harnstoff, N,N'-Bis-(2-hydroxyethyl)harnstoff, N-(3-Hydroxypropyl)harnstoff, N,N-Bis-(3-hydroxypropyl)harnstoff, N,N'-Bis-(3-hydroxypropyl)harnstoff, N-(2-Hydroxypropyl)harnstoff, N,N-Bis-(2-hydroxypropyl)harnstoff, N,N'-Bis-(2-hydroxypropyl)-harnstoff, N-(2-Hydroxy-2-methyl-prop-2-yl)harnstoff, N,N-Bis-(2-hydroxy-2-methyl-prop-2-yl)harnstoff, N,N'-Bis-(2-hydroxy-2-methyl-prop-2-yl)harnstoff, N-(1,3-Dihydroxy-2-methyl-prop-2-yl)harnstoff, N,N-Bis-(1,3-dihydroxy-2-methyl-prop-2-yl)harnstoff, N,N'-Bis-(1,3-dihydroxy-2-methyl-prop-2-yl)harnstoff, N-(1,3-Dihydroxy-2-hydroxymethyl-prop-2-yl)harnstoff, N,N-Bis-(1,3-dihydroxy-2-hydroxymethyl-prop-2-yl)harnstoff und N,N'-Bis-(1,3-dihydroxy-2-hydroxymethyl-prop-2-yl)harnstoff ausgewählt. Ganz besonders bevorzugt ist N-(2-Hydroxyethyl)harnstoff und N,N'-Bis-(2-hydroxyethyl)harnstoff, erhältlich beispielsweise als Handelsprodukt Hydrovance^{®} von der Firma National Starch.

Erfindungsgemäß verwendete kosmetische Mittel enthalten Harnstoff-Derivate der zuvor genannten Formel (I) bevorzugt in Mengen von 0,75 bis 4,5 Gew.-%, mehr bevorzugt von 1 bis 4 Gew.-%, besonders bevorzugt von 2 bis 3,5 Gew.-% und insbesondere von 2,5 bis 3,2 Gew.-%, wobei sich die Mengenangaben auf das Gesamtgewicht des Mittels beziehen.

Geeignete Vitamine, Provitamine oder Vitaminvorstufen der Vitamingruppen B, C, E, H und K können beispielsweise ausgewählt sein aus:
i) Vitamin B: Zur Vitamin B-Gruppe oder zu dem Vitamin B-Komplex gehören unter anderem
   - Vitamin B₁: Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in den erfindungsgemäß verwendeten Mitteln in Mengen von 0,05 bis 1 Gew.-%, bezogen auf das gesamte Mittel, eingesetzt,
   - Vitamin B₂: Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in den erfindungsgemäß verwendeten Mitteln in Mengen von 0,05 bis 1 Gew.-%, bezogen auf das gesamte Mittel, eingesetzt,
   - Vitamin B₃: Unter dieser Bezeichnung werden die Verbindungen Nicotinsäure und Nicotinsäureamid (Niacinamid) geführt. Bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten sein kann,
   - Vitamin B₅: Pantothensäure und Panthenol. Einsetzbare Derivate des Panthenols sind insbesondere die Ester und Ether des Panthenols sowie kationisch derivatisierte Panthenole. An Stelle von sowie zusätzlich zu Pantothensäure oder Panthenol können die erfindungsgemäß verwendeten Mittel auch Derivate des 2-Furanon mit der allgemeinen Strukturformel (II) enthalten, 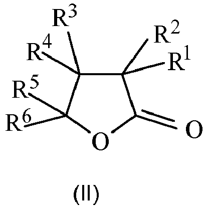 wobei 2-Furanon-Derivate bevorzugt sind, in denen die Substituenten R¹ bis R⁶ unabhängig voneinander ein Wasserstoffatom, einen Hydroxylrest, einen Methyl- , Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasser stoffrest oder einen gesättigten oder ein- oder zweifach ungesättigten, verzweig ten oder linearen Mono-, Di- oder Triamino-C₂-C₄ - Kohlenwasserstoffrest dar stellen.
      Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Ein außerordentlich bevorzugtes 2-Furanon-Derivat ist Pantolacton (Dihydro-3-hydroxy-4,4-dimethyl-2(3H)-furanon), wobei in Formel (II) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
      Die genannten Verbindungen des Vitamin B₅-Typs sowie die 2-Furanonderivate können in den erfindungsgemäß verwendeten Mitteln in einer Gesamtmenge von 0,05 bis 5 Gew.-%, bevorzugt von 0,1 bis 3 Gew.-%, besonders bevorzugt von 0,5 bis 2 Gew.-%, jeweils bezogen auf das gesamte Mittel, enthalten sein,
   - Vitamin B₆: hierunter wird keine einheitliche Substanz, sondern die unter den Trivialnamen Pyridoxin, Pyridoxamin und Pyridoxal bekannten Derivate des 5-Hydroxymethyl-2-methylpyridin-3-ols verstanden. Vitamin B₆ kann in den erfindungsgemäß verwendeten Mitteln bevorzugt in Mengen von 0,0001 bis 1,0 Gew.-%, insbesondere in Mengen von 0,001 bis 0,01 Gew.-%, enthalten sein, wobei sich die Mengenangaben auf das Gesamtgewicht des Mittels beziehen,
   - Vitamin B₇: (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin kann in den erfindungsgemäß verwendeten Mitteln bevorzugt in Mengen von 0,0001 bis 1,0 Gew.-%, insbesondere in Mengen von 0,001 bis 0,01 Gew.-% enthalten sein, wobei sich die Mengenangaben auf das Gesamtgewicht des Mittels beziehen,
(ii) Vitamin C (Ascorbinsäure): kann in den erfindungsgemäß verwendeten Mitteln bevorzugt in Mengen von 0,1 bis 3 Gew.-%, bezogen auf das gesamte Mittel, eingesetzt werden. Die Verwendung der Derivate Ascorbylpalmitat, - stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein,
(iii) Vitamin E: Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate können in den erfindungsgemäß verwendeten Mitteln bevorzugt in Mengen von 0,05 - 1 Gew.-%, bezogen auf das gesamte Mittel, enthalten sein,
(iv) Vitamin F: Unter Vitamin F werden üblicherweise essentielle Fettsäuren, insbesondere Linolsäure, Linolensäure und Arachidonsäure, verstanden,
(v) Vitamin H: Vitamin H ist eine andere Bezeichnung für Biotin oder Vitamin B₇ (siehe oben),
(vi) Vitamin K: Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K kann in den erfindungsgemäß verwendeten Mitteln bevorzugt in Mengen von 0,0001 bis 1,0 Gew.-%, insbesondere 0,01 bis 0,5 Gew.-%, jeweils bezogen auf das gesamte Mittel, enthalten sein.

Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, Panthenol und Pantolacton sind besonders bevorzugt.

Geeignete Monomere von Aminosäuren und/oder der N-C₂-C₂₄-Acylaminosäuren können ausgewählt sein aus Alanin, Arginin, Asparagin, Asparaginsäure, Canavanin, Citrullin, Cystein, Cystin, Desmosin, Glutamin, Glutaminsäure, Glycin, Histidin, Hydroxylysin, Hydroxyprolin, Isodesmosin, Isoleucin, Leucin, Lysin, Methionin, Ornithin, Phenylalanin, Prolin, Pyroglutaminsäure, Sarcosin, Serin, Taurin, Threonin, Thyroxin, Tryptophan, Tyrosin und Valin. Besonders bevorzugt sind Taurin, Arginin, Hydroxyprolin, Prolin und Glutaminsäure.
Der C₂ - C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Bevorzugte Beispiele sind Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Zinkpyroglutamat und Natriumlauroylglutamat.
Physiologisch verträgliche Salze der zuvor genannten Wirkstoffe, die Säuregruppen enthalten und Salze bilden können, können ausgewählt sein aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.
Geeignete Oligomere von Aminosäuren und/oder der N-C₂-C₂₄-Acylaminosäuren können ausgewählt sein aus Di-, Tri-, Tetra-, Penta-, Hexa-, Hepta-, Octa-, Nona- und Decapeptiden, die acyliert und/oder verestert sein können. Besonders bevorzugt sind die Di-, Tri-, Tetra-, Penta- und Hexapeptide, die acyliert und/oder verestert sein können. Bevorzugte, gegebenenfalls acylierte und/oder veresterte Dipeptide sind Tyr-Arg, Val-Trp, Asn-Phe, Asp-Phe, N-Palmitoyl-ß-Ala-His, N-Acetyl-Tyr-Arg-hexyldecylester (z.B. Calmosensine von Sederma), Carnosin (ß-Ala-His) und N-Palmitoyl-Pro-Arg. Bevorzugte, gegebenfalls acylierte und/oder veresterte Tripeptide sind Gly-His-Lys, N-Palmitoyl-Gly-His-Lys, Gly-Lys-His, His-Ala-Orn, Lys-Phe-Lys, N-Elaidoyl-Lys-Phe-Lys und N-Acetyl-Arg-Lys-Arg-NH₂. Bevorzugte, gegebenenfalls acylierte und/oder veresterte Tetrapeptide sind Gly-Gln-Pro-Arg, Gly-Gln-Arg-Pro und N-Palmitoyl-Gly-Gln-Pro-Arg. Bevorzugte, gegebenenfalls acylierte und/oder veresterte Pentapeptide sind Lys-Thr-Thr-Lys-Ser, N-Palmitoyl-Lys-Thr-Thr-Lys-Ser, N-Palmitoyl-Tyr-Gly-Gly-Phe-Met und N-Palmitoyl-Tyr-Gly-Gly-Phe-Leu. Ein bevorzugtes Hexapeptid ist Palmitoyl-Val-Gly-Val-Ala-Pro-Gly (Biopeptide EL von Sederma).
Es kann besonders bevorzugt sein, dass die erfindungsgemäß verwendeten Mittel ein Gemisch aus mindestens zwei Oligopeptiden enthalten. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z.B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich.

Geeignete Polymere von Aminosäuren und/oder der N-C₂-C₂₄-Acylaminosäuren können ausgewählt sein aus pflanzlichen und tierischen Proteinhydrolysaten und/oder Proteinen. Unter tierischen Proteinhydrolysaten sind z. B. Elastin-, Collagen-, Keratin-, Seiden- und Milcheiweiß-Proteinhydrolysate zu verstehen, die auch in Form von Salzen vorliegen können. Bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine^{®} von Coletica oder Ridulisse C^{®} von Silab.
Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Erfindungsgemäß einsetzbar sind auch kationisierte Proteinhydrolysate.
In einer weiteren bevorzugten Ausführungsform können die Polymeren der Aminosäuren ausgewählt seinaus DNA-Reparaturenzymen.
Bevorzugte DNA-Reparaturenzyme sind Photolyase und T4 Endonuclease V, letztere im Weiteren mit "T4N5" abgekürzt. Diese beiden Enzyme sind im Stand der Technik bereits als so genannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.
In den erfindungsgemäß verwendeten Mitteln können die Photosome™ oder Ultrasome™ bevorzugt in Mengen von 0,1 - 10 Gew.-%, mehr bevorzugt von 0,5 - 5,0 Gew.-% und besonders bevorzugt von 1,0 - 4,0 Gew.-%, bezogen auf das gesamte Mittel, enthalten sein.
In den erfindungsgemäß verwendeten Mitteln können die Monomere, Oligomere oder Polymere von Aminosäuren, N-C₂-C₂₄-Acylaminosäuren und/oder den Estern und/oder den physiologisch verträglichen Metallsalzen dieser Substanzen bevorzugt in Mengen von 0,0001 - 10 Gew.-%, mehr bevorzugt von 0,01 - 5 Gew.-% und besonders bevorzugt von 0,1 - 3 Gew.-%, jeweils bezogen auf das gesamte Mittel, enthalten sein.

Unter geeigneten DNA- oder RNA-Oligonucleotiden werden bevorzugt Polymerisate aus 2 bis 20, mehr bevorzugt aus 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).
Ein besonders bevorzugtes Oligonucleotid ist das Thymidin-Dinucleotid.
In den erfindungsgemäß verwendeten Mitteln können die DNA-Oligonucleotide oder RNA-Oligonucleotide vorzugsweise in Mengen von 0,0001 - 5 Gew.-%, bevorzugt von 0,001 - 1,0 Gew.-% und besonders bevorzugt von 0,01 - 0,5 Gew.-%, bezogen auf das gesamte Mittel, enthalten sein.

Geeignete natürliche Betainverbindungen sind bevorzugt natürlich vorkommende Verbindungen mit der Atomgruppierung R₃N⁺-CH₂-X-COO⁻ gemäß IUPAC-Regel C-816.1. So genannte Betaintenside (synthetisch) fallen nicht unter die geeigneten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).
Die Betainverbindungen können in den erfindungsgemäß verwendeten Mitteln vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf das gesamte Mittel, enthalten sein.

Unter geeigneten α-Hydroxycarbonsäuren, α-Ketocarbonsäuren oder β-Hydroxycarbonsäuren oder deren Ester-, Lacton- oder Salzformen werden bevorzugt Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure verstanden. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren können ausgewählt sein aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate können in den erfindungsgemäß verwendeten Mitteln vorzugsweise in Mengen von 0,1 - 10 Gew.-%, bevorzugt von 0,5 - 5 Gew.-% enthalten sein, wobei sich die Mengenangaben auf das gesamte Mittel beziehen.

Bevorzugte Flavonoide umfassen die Glycoside der Flavone, der Flavanone, der 3-Hydroxyflavone (Flavonole), der Aurone und der Isoflavone. Besonders bevorzugte Flavonoide können ausgewählt sein aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid). Außerordentlich bevorzugte Flavonoide sind α-Glucosylrutin, Naringin und Apigenin-7-glucosid. Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.
Die Flavonoide können in den erfindungsgemäß verwendeten Mitteln vorzugsweise in Mengen von 0,0001 bis 1 Gew.-%, bevorzugt von 0,0005 bis 0,5 Gew.-% und besonders bevorzugt von 0,001 bis 0,1 Gew.-% enthalten sein, jeweils bezogen auf die Flavonoidaktivsubstanz in dem gesamten kosmetischen Mittel.
Zu den geeigneten Isoflavonoiden werden bevorzugt die Isoflavone und die Isoflavon-Glycoside gezählt.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht.

Besonders bevorzugte Isoflavon-Glycoside sind Daidzin und Genistin.

Weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt können Isoflavone oder Isoflavon-Glycoside in Form von aus Soja gewonnenen Extrakten in den erfindungsgemäß verwendeten Mitteln eingesetzt werden, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Die Isoflavonoide können in den erfindungsgemäß verwendeten Mitteln vorzugsweise in Mengen von 0,00001 bis 1 Gew.-%, bevorzugt von 0,0005 bis 0,5 Gew.-% und besonders bevorzugt von 0,001 bis 0,1 Gew.-% enthalten sein, jeweils bezogen auf die Isoflavonoidaktivsubstanz in dem gesamten kosmetischen Mittel.

In einer weiteren bevorzugten Ausführungsform können die erfindungsgemäß verwendeten Mittel zusätzlich mindestens ein Polyphenol oder einen Polyphenol-reichen Pflanzenextrakt enthalten. Unter Polyphenolen sind bevorzugt aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.
Die Polyphenole können in den erfindungsgemäß verwendeten Mitteln vorzugsweise in Mengen von 0,001 bis 10 Gew.-%, bevorzugt von 0,005 bis 5 Gew.-% und besonders bevorzugt von 0,01 bis 3 Gew.-%, jeweils bezogen auf das gesamte kosmetische Mittel, enthalten sein.

In einer weiteren bevorzugten Ausführungsform können die erfindungsgemäß verwendeten Mittel weiterhin mindestens ein Ubichinon oder ein Ubichinol oder deren Derivate enthalten. Ubichinole sind die reduzierte Form der Ubichinone. Bevorzugte Ubichinone weisen die Formel (III) auf: 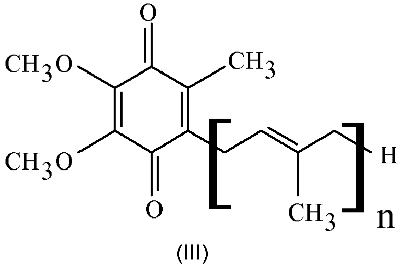 mit n = 6, 7, 8, 9 oder 10.
Besonders bevorzugt ist das Ubichinon der Formel (III) mit n = 10, auch bekannt als Coenzym Q10.
Die Ubichinone, Ubichinole oder deren Derivate können in den erfindungsgemäß verwendeten Mitteln vorzugsweise in Mengen von 0,0001 bis 1 Gew.-%, bevorzugt von 0,001 bis 0,5 Gew.-% und besonders bevorzugt von 0,005 bis 0,1 Gew.-%, jeweils bezogen auf das gesamte Mitte, enthalten sein.

Silymarin stellt ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.
Silymarin kann in den erfindungsgemäß verwendeten Mitteln vorzugsweise in Mengen von 0,0001 bis 1 Gew.-%, bevorzugt von 0,001 bis 1,0 Gew.-% und besonders bevorzugt von 0,005 bis 0,5 Gew.-%, jeweils bezogen auf das gesamte Mittel, enthalten sein.

In einer weiteren bevorzugten Ausführungsform können die erfindungsgemäß verwendeten Mittel mindestens ein natürlich vorkommendes Xanthin-Derivat, ausgewählt aus Coffein, Theophyllin, Theobromin und Aminophyllin, enthalten. Besonders bevorzugt ist Coffein.

Die natürlich vorkommenden Xanthin-Derivate können in den erfindungsgemäß verwendeten Mitteln vorzugsweise in Mengen von 0,0001 bis 5 Gew.-%, bevorzugt von 0,001 bis 3 Gew.-% und besonders bevorzugt von 0,005 bis 1 Gew.-%, jeweils bezogen auf das gesamte Mittel, enthalten sein.

Ectoin ist der Trivialname für 2-Methyl-1,4,5,6-tetrahydropyrimidin-4-carboxylat. Ectoin kann in den erfindungsgemäß verwendeten Mitteln vorzugsweise in Mengen von 0,0001 bis 1 Gew.-%, bevorzugt von 0,001 bis 0,5 Gew.-% und besonders bevorzugt von 0,005 bis 0,01 Gew.-%, jeweils bezogen auf das gesamte Mittel, enthalten sein.

Bei geeigneten UV-Filtersubstanzen handelt es sich bevorzugt um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist bevorzugt.
Besonders geeignete UV-Filter können ausgewählt sein aus den physiologisch verträglichen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone, Uvinul^{®} T 150), Dimethicodiethylbenzal malonate (CAS-Nr. 207574-74-1, Parsol^{®} SLX), Dioctyl Butamido Triazone (Uvasorb^{®} HEB), 2,4-bis-[5-1(dimethylpropyl)benzoxazol-2-yl-(4-phenyl)-imino]-6-(2-ethylhexyl)-imino-1,3,5-triazin (CAS Nr. 288254-16-0, Uvasorb^{®} K2A) und sowie beliebige Mischungen der genannten Komponenten. Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bevorzugte anorganische Lichtschutzpigmente sind feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.
Die organischen UV-Filtersubstanzen können in den erfindungsgemäß verwendeten Mitteln bevorzugt in Mengen von 0,01 - 30 Gew.-%, mehr bevorzugt von 0,05 - 20 Gew.-%, besonders bevorzugt von 0,1 - 15 Gew.-% und außerordentlich bevorzugt von 0,25 - 10 Gew.-%, jeweils bezogen auf das gesamte Mittel, enthalten sein.
Die anorganischen UV-Filtersubstanzen können in den erfindungsgemäß verwendeten Mitteln vorzugsweise in Mengen von 0,01 - 15 Gew.-%, bevorzugt von 0,05 - 10 Gew.-%, besonders bevorzugt von 0,1 - 5 Gew.-% und außerordentlich bevorzugt von 0,25 - 4,0 Gew.-%, jeweils bezogen auf das gesamte Mittel, enthalten sein.

Geeignete hautaufhellende Wirkstoffe können ausgewählt sein aus Ascorbinsäure, den Estern der Ascorbinsäure mit Phosphorsäure und/oder organischen C₂C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt. Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat.

Die hautaufhellenden Wirkstoffe können in den erfindungsgemäß verwendeten Mitteln vorzugsweise in einer Menge von 0,05 bis 5 Gew.-%, bevorzugt von 0,1 - 2 Gew-%, jeweils bezogen auf das gesamte Mittel, enthalten sein.

Geeignete hautberuhigende Wirkstoffe sind bevorzugt ausgewählt aus Farnesol, Allantoin, α-Bisabolol und α-Liponsäure.
Die hautberuhigenden Wirkstoffe können in den erfindungsgemäß verwendeten Mitteln vorzugsweise in Mengen von 0,001 bis 5 Gew.-%, bevorzugt von 0,005 bis 1 Gew.-%, jeweils bezogen auf das gesamte Mittel, enthalten sein.

Geeignete sebumregulierende Wirkstoffe können ausgewählt sein aus Acnacidol, Azelainsäure, Azeloglycina und einem Extrakt aus Spiraea Ulmaria. Der Extrakt ist z.B. im Produkt Seburegul der Firma Silab enthalten. Die sebumregulierenden Wirkstoffe können in den erfindungsgemäß verwendeten Mitteln vorzugsweise in Mengen von 0,001 bis 5 Gew.-%, bevorzugt 0,01 bis 2 Gew.-% und besonders bevorzugt 0,1 bis 1 Gew.-%, jeweils bezogen auf das gesamte Mittel, enthalten sein.

In einer besonders bevorzugten Ausführungsform wird Climbazol in kosmetischen Hautbehandlungsmitteln verwendet, die neben dem zuvor beschriebenen Träger bevorzugt mindestens zwei, mehr bevorzugt mindestens drei und insbesondere bevorzugt mindestens vier Wirkstoffe aus der zuvor genannten Gruppe enthalten. Insbesondere bevorzugt ist die erfindungsgemäße Verwendung in Hautbehandlungsmitteln, die als weitere Wirkstoffe mindestens zwei Wirkstoffe aus der Gruppe der Vitamine (wie zuvor beschrieben), der Harnstoff-Derivate nach Formel (I), der natürlichen Betainverbindungen, der Xanthin-Derivate und/oder der hautberuhingenden Wirkstoffe enthalten.

Erfindungsgemäß verwendete Mittel können auch als Pasten, Salben, Lotionen oder Cremes vorliegen. Feste Mittel können beispielsweise als loser Puder, gepresster Puder oder als Stift vorliegen.

Vorteilhafterweise liegen die erfindungsgemäß verwendeten Hautbehandlungsmittel in Form einer flüssigen, fliessfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

Besonders bevorzugte erfindungsgemäß verwendete Mittel liegen in Form einer fließfähigen Emulsion vor und enthalten bevorzugt mindestens einen weiteren hautkonditionierenden Wirkstoff und mindestens einen Emulgator.

Unter geeigneten konditionierenden Wirkstoffen sind bevorzugt solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Bevorzugte konditionierende Wirkstoffe können ausgewählt sein aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsaeuren, besonders linearen und/oder verzweigten, gesaettigten und/oder ungesaettigten C₈₋₃₀-Fettsaeuren, Fettalkoholen, besonders gesättigten, ein- oder mehrfach ungesättigten, verzweigten oder unverzweigten Fettalkoholen mit 4-30 Kohlenstoffatomen, die mit 1-75, bevorzugt 5-20 Ethylenoxid-Einheiten ethoxyliert und/oder mit 3-30, bevorzugt 9-14 Propylenoxid-Einheiten propoxyliert sein können, Esterölen, das heißt Estern von C₆₋₃₀-Fettsaeuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsaeureestern von gesättigten und/oder ungesättigten linearen und/oder verzweigten Fettsäuren mit Glycerin, die mit 1-10, bevorzugt 7- 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsaeuren, z. B. gehärteten Triglyceridfetten, Phospholipiden, beispielsweise Sojalecithin, Ei-Lecithin und Kephalinen, Shea-Butter, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin-und/oder Hydroxy-Gruppen enthalten, bevorzugt Substanzen mit den INCI-Bezeichnungen Dimethiconol, Amodimethicone oder Trimethylsilylamodimethicone. Die Einsatzmenge der Fettstoffe in den erfindungsgemäß verwendeten Mitteln beträgt bevorzugt 0,1-99 Gew.-%, besonders bevorzugt 2-50 Gew.-% und außerordentlich bevorzugt 5-20 Gew.-%, jeweils bezogen auf das gesamte Mittel.

Geeignete oberflächenaktive Substanzen und/oder Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsaeuren und an C₈-C₁₅-Alkylphenole, C₁₂-C₂₂-Fettsaeuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und - oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®}68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsaeuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsaeureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsaeuren und deren Na-, K-, Ammonium-, Ca-, Mg-und Zn-Salze.

Die erfindungsgemäß verwendeten Mittel können die Emulgatoren bevorzugt in Mengen von 0,1 bis 25 Gew.-%, besonders bevorzugt 0,5-15 Gew.-%, bezogen auf das gesamte Mittel, enthalten.

Weitere geeignete Zusatzstoffe der Emulsionen sind Verdickungsmittel, z. B. anionische Polymere aus Acrylsäure, Methacrylsäure, Crotonsäure, Maleinsäureanhydrid und 2-Acrylamido-2-methylpropansulfonsaeure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäeure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS und Simulgel^{®} EPG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80-98% eine ungesättigte, gewünschtenfalls substituierte C₃₋₆-Carbonsaeure oder ihr Anhydrid sowie zu 2-20% gewünschtenfalls substituierte Acrylsäureester von gesättigten C₁₀₋₃₀-Carbonsaeuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Weitere geeignete Zusatzstoffe sind beispielsweise:
- Parfumöle,
- Substanzen zur Einstellung des pH-Wertes,
- Komplexbildner wie EDTA, NTA, β-Alanindiessigsaeure und Phosphonsaeuren,
- Treibmittel wie Propan-Butan-Gemische, Pentan, Isopentan, Isobutan, N₂O, Dimethylether, CO₂ und Luft,
- Wirkstoffe wie Hyaluronsäure und/oder Glycyrrhizinsäure bzw. deren physiologisch verträglich Salze und/oder Derivate,
- Pigmente und/oder
- Abrasivstoffe.

Die Verwendung von Climbazol in kosmetischen Mitteln, insbesondere in kosmetischen Hautbehandlungsmitteln, weist den Vorteil auf, dass sie zu einer Stimulierung der Kollagenneusynthese in der Dermis führt.
Climbazol ist demnach geeignet als RAMBA.
Die Haut weist nach der Behandlung mit Climbazol in einem topischen Träger ein verfeinertes Hautbild auf.
Weiterhin lässt sich durch die erfindungsgemäße Verwendung die Hautfestigkeit verbessern - die Haut wird insgesamt gestrafft.
Zudem konnten Hautfalten sichtbar reduziert werden (2 von 3 Falten werden reduziert).
Durch die Stimulation der Kollagen- und/oder Hualuronsäuresynthese in der Dermis können die Anzeichen dermalen Gewebeschwundes beseitigt und/oder vermindert werden.

Ein zweiter Gegenstand der Erfindung ist ein demnach ein kosmetisches, nicht-therapeutisches Verfahren zur Verbesserung der Hautfestigkeit und/oder zur Hautstraffung und/oder zur Reduktion von Hautfalten, bei dem Climbazol in einem geeigneten kosmetischen Träger mit der Haut in Kontakt gebracht wird.

Ein dritter Gegenstand der Erfindung ist ein demnach ein kosmetisches, nicht-therapeutisches Verfahren zur Verbesserung dermalen Gewebeschwundes, bei dem Climbazol in einem geeigneten kosmetischen Träger mit der Haut in Kontakt gebracht wird.

Erfindungsgemäß bevorzugt ist ein kosmetisches Verfahren, bei dem Climbazol bevorzugt in einer Menge von 0,001 bis 2 Gew.-%, mehr bevorzugt von 0,0025 bis 1,5 Gew.-%, und insbesondere von 0,005 bis 1 Gew.-% in dem kosmetischen Träger enthalten ist, wobei sich die Mengenangaben auf das Gesamtgewicht des kosmetischen Trägers beziehen.

Erfindungsgemäß weiterhin bevorzugt ist ein kosmetisches Verfahren, bei dem durch die Verwendung von Climbazol die Kollagen- und/oder die Hyaloronsäuresynthese in der Dermis beeinflusst, bevorzugt stimuliert und/oder verbessert wird.

Hinsichtlich des kosmetischen Trägers und der weiteren fakultativen Inhaltsstoffe des kosmetischen Trägers gilt das unter den Abschnitten zur erfindungsgemäßen Verwendung Gesagte.

### Beispiele:

### 1) Identifikation des Climbazols als RAMBA

Verschiedene kosmetische Wirkstoffe, die zur Stoffklasse der Azole gerechnet werden können, wurden auf eine mögliche RAMBA-Aktivität hin untersucht.
Zu diesem Zweck wurden humane Keratinocyaten für 16 Stunden mit den entsprechenden Substanzen in Kombination mit Retinsäure inkubiert und anschließend mittels qPCR die Expressionsänderung des Markers CYP26A1 verfolgt. Das Enzym CYP26A1 bewirkt die Metabolisierung der Retinsäure und wird durch die Retinsäure selber induziert, wodurch eine Feedbak-Regulation der Retinsäurekonzentration in der Zelle ermöglicht wird. Bei gleichzeitiger Applikation eines RAMBAS ist das Ausmaß dieser Inkubation deutlich geringer, und somit der Retinsäureabbau gehemmt. Als Kontrolle wurde das bekannte RAMBA Ketokonazol eingesetzt.

**Tabelle 1**

| | Rel. Expression CYP26A1 | STD |
|---|---|---|
| Kontrolle 96% Ethanol | 1,0 | 0,3 |
| Retinsäure (RA) [10nM] | 37,6 | 0,3 |
| RA + Ketokonazol [10µM] | 11,1 | 0,1 |
| RA + Climbazol [0,001 %] | 26,6 | 0,1 |

Tabelle 1 zeigt, dass Climbazol (ebenso wie Ketokonazol) bei der Kombination mit Retinsäure die Induktion von CYP26A1 deutlich vermindert.

Climbazol zeigt demnach in etwa die gleiche RAMBA-Aktivität wie Ketokonazol.

### 2) Nachweis der Kollagenneusynthese

Ein in der Kosmetik relevanter Parameter für die Anti-Aging-Leistung eines Produktes ist die Induktion der Kollagen-Neusynthese. Für Retinsäure wurde die Induktion der Kollagensynthese bereits beschrieben.

Zum Nachweis der Kollagen-Neusynthese wurde ein Co-Kultur-System verwendet, das aus einer zweidimensionalen Fibroblasten-Kultur besteht, auf die in einem Insert ein dreidimensionales Epidermismodell aufgesetzt wurde. Über das Nährmedium kann ein Stoffaustausch zwischen den Fibroblasten und den Keratinocyten des Epidermismodells erfolgen.

Das Co-Kultur-System wurde mit den RAMBA-Wirkstoffen Ketokonazol und Climbazol behandelt und die Kollagen-Neusynthese über die Menge des in dem Medium sekretierten P1 NP, dem aminoterminalen Propeptid des Typ-I-Kollagens, bestimmt. Als Kontrollen für die Syntheseinduktion wurden Retinsäure und Retinol eingesetzt.

**Tabelle 2**

| | P1 NP (µg/ml) | STD |
|---|---|---|
| unbehandelt | 107 | 18 |
| Retinsäure [5µM] | 200 | 37 |
| Retinol [5µM] | 146 | 4 |
| Ketokonazol [50µM] | 196 | 57 |
| Climbazol [0,005%] | 135 | 14 |

Ketokonazol und Climbazol zeigen eine Induktion der Kollagensynthese, die auf einen verminderten Abbau der endogenen Retinsäure zurückzuführen ist.

### 3) Ausführungsbeispiele für die erfindungsgemäße Verwendung des 3-Methyl-1-phenyl-5-pyrazolons in Gesichtscremes

**Tabelle 3**

| | Beispiel 1 [Gew.-%] | Beispiel 2 [Gew.-%] |
|---|---|---|
| Montanov 202^{®1} | 5 | 5 |
| Capryl-/Caprinsäure Triglycerid | 7 | 7 |
| Cetiol CC^{®2} | 5 | 5 |
| Novata AB PH^{®3} | 2 | 2 |
| DC EL-8040 ID^{®4} | 0,9 | 1 |
| Tocopherolacetat | 0,5 | 0,3 |
| Cetiol SB 45^{®5} | 1,5 | 2 |
| Coffein | 0,3 | |
| Glycerin | 5 | 5 |
| Hexandiol-1,6 | 5 | 5 |
| Betafin BP 20^{®6} | 2 | 2 |
| Tego Carbomer 140^{®7} | 0,4 | 0,35 |
| Hydrovance^{®8} | 2,8 | 3 |
| Climbazol | 0,05 | 0,075 |
| Panthenol 75% | 0,75 | |
| Bisabolol | | 0,05 |
| Matrixyl 3000^{®9} | 3 | 3 |
| Simulgel EPG^{®10} | 1 | 1 |
| Parfum, Konservierungsmittel | q.s. | q.s. |
| Wasser | ad 100 | ad 100 |

In Tabelle 3 wurden die folgenden Handelsprodukte eingesetzt:
1 INCI-Bezeichnung: Arachidyl Alcohol, Behenyl Alcohol, Arachidyl Glucoside; Seppic
2 INCI-Bezeichnung: Dicaprylyl Carbonate; BASF
3 INCI-Bezeichnung: Coco Glycerides; BASF
4 INCI-Bezeichnung: Isododecane, Dimethicone Crosspolymer; Dow Corning
5 INCI-Bezeichnung: Shea Butter (Butyrospermum Parkii); BASF
6 INCI-Bezeichnung: Trimethylglycine; Finnfeeds Finnland Oy
7 INCI-Bezeichnung: Carbomer; Evonik
8 INCI-Bezeichnung: Hydroxyethyl Urea; Akzo Nobel
9 INCI-Bezeichnung: Glycerin, Aqua (Water), Butylene Glycol, Carbomer, Coco Glucoside, Palmitoyl Oligopeptide, Palmitoyl Tetrapeptide-7; Sederma,
10 INCI-Bezeichnung: Sodium Acrylate/Sodium Acryloyldimethyl Taurate Copolymerf, Polyisobutene, Caprylyl/Capryl Glucoside; Seppic

## Patentansprüche

1. Kosmetische, nicht-therapeutische Verwendung von Climbazol (Imidazolyl-1-(4-chlorophenoxy)-3,3-dimethylbutane-2-one) zur Beeinflussung der natürlichen Produktion von Bestandteilen in der Dermis.

2. Verwendung nach Anspruch 1, **dadurch gekennzeichnet, dass** die natürliche Produktion von Bestandteilen der extrazellulären Matrix in der Dermis beeinflusst, insbesondere stimuliert wird.

3. Verwendung nach Anspruch 1 oder 2, **dadurch gekennzeichnet, dass** die natürliche Produktion von Kollagen, Elastin und/oder Glycosaminglykanen in der extrazellulären Matrix beeinflusst, bevorzugt stimuliert und/oder verbessert, wird.

4. Verwendung nach Anspruch 3, **dadurch gekennzeichnet, dass** die Kollagen- und/oder die Hyaluronsäuresynthese beeinflusst, bevorzugt stimuliert und/oder verbessert wird.

5. Verwendung nach einem der Ansprüche 1 bis 4, **dadurch gekennzeichnet, dass** die Verwendung topisch erfolgt.

6. Verwendung nach einem der Ansprüche 1 bis 5, **dadurch gekennzeichnet, dass** 0,001 bis 2 Gew.-%, bevorzugt von 0,0025 bis 1,5 Gew.-%, und insbesondere von 0,005 bis 1 Gew.-% Climbazol in einem kosmetischen Hautbehandlungsmittel enthalten sind, wobei sich die Mengenangaben auf das Gesamtgewicht des kosmetischen Hautbehandlungsmittels beziehen.

7. Kosmetisches, nicht-therapeutisches Verfahren zur Verbesserung der Hautfestigkeit und/oder zur Hautstraffung und/oder zur Reduktion von Hautfalten, **dadurch gekennzeichnet, dass** man Climbazol in einem geeigneten kosmetischen Träger mit der Haut in Kontakt bringt.

8. Kosmetisches, nicht-therapeutisches Verfahren zur Verbesserung dermalen Gewebeschwundes, **dadurch gekennzeichnet, dass** man Climbazol in einem geeigneten kosmetischen Träger mit der Haut in Kontakt bringt.

9. Kosmetisches Verfahren nach einem der Ansprüche 7 oder 8, **dadurch gekennzeichnet, dass** Climbazol in einer Menge von 0,001 bis 2 Gew.-%, bevorzugt von 0,0025 bis 1,5 Gew.-%, und insbesondere von 0,005 bis 1 Gew.-% in dem kosmetischen Träger enthalten ist, wobei sich die Mengenangabe auf das Gesamtgewicht des kosmetischen Trägers bezieht.

10. Kosmetisches Verfahren nach einem der Ansprüche 7 bis 9, **dadurch gekennzeichnet, dass** die Kollagen- und/oder die Hyaluronsäuresynthese in der Dermis beeinflusst, bevorzugt stimuliert und/oder verbessert wird.
